# EUROPEAN PATENT SPECIFICATION

(11) **EP 4 458 590 B1**
(45) Date of publication and mention of the grant of the patent: **01.04.2026**
(21) Application number: 22915469.5
(22) Date of filing: 17.10.2022
(51) Int. Cl.: B60C 19/12, B60C 11/02, B60C 19/00, G01N 17/00, G06Q 10/20, G06Q 50/10, B29C 73/10, B60C 13/00, B60C 11/24, G06Q 50/40, G01N 33/44, B60C 23/20

(54) **REPAIR PATCH AND PRECURED TREAD**
REPARATURPFLASTER UND VORGEHÄRTETE LAUFFLÄCHE
PLAQUE DE RÉPARATION ET BANDE DE ROULEMENT PRÉDURCIE

(30) Priority: 27.12.2021 JP 2021213470; 27.12.2021 JP 2021213472; 27.12.2021 JP 2021213473
(43) Date of publication of application: 06.11.2024
(73) Proprietor: BRIDGESTONE CORPORATION, Chuo-ku Tokyo 104-8340 (JP)
(72) Inventor: YAMAMOTO Taku, Tokyo 104-8340 (JP); MORIGUCHI Akira, Tokyo 104-8340 (JP)
(74) Representative: Marks & Clerk LLP
(86) International application number: PCT/JP2022/038645
(87) International publication number: WO 2023/127234

(56) References cited:
- EP-A1- 3 495 166
- WO-A1-2017/203850
- JP-A- 2004 508 998
- JP-A- 2004 508 998
- JP-A- 2006 273 260
- JP-A- 2009 107 484
- JP-A- 2010 179 824
- JP-A- 2010 179 824
- JP-A- 2011 057 203
- JP-A- 2011 057 203
- JP-A- 2012 046 027
- JP-A- 2012 046 027
- JP-A- 2012 254 655
- JP-A- 2012 254 655
- JP-A- 2014 144 774
- JP-A- 2014 144 774
- JP-A- 2017 039 360
- JP-A- 2017 039 360
- JP-A- 2021 095 120
- JP-A- 2021 095 120
- KR-A- 20180 003 147
- US-A- 5 358 772
- US-A1- 2003 047 264
- US-A1- 2006 042 366
- US-A1- 2015 269 468
- US-A1- 2017 158 002
- US-A1- 2020 300 773

## Description

### TECHNICAL FIELD

The present invention relates to a repair patch and a pre-cured tread.

### BACKGROUND

In recent years, with the development of the sharing economy in the transportation industry and the increasing use of retreaded tires, technology for understanding the degradation state of tires has been attracting attention. For example, Patent Literature (PTL) 1 discloses a tire whose usage limit is clearly indicated by a difference in the degree of discoloration between a character portion and a background portion when a display portion is exposed to light.

### CITATION LIST

### Patent Literature

PTL 1: JP 2006-273260 A
PTL 2: US 2017158002 A1 discloses a repair patch for an elastomeric component, in particular for a vehicle tire, to a use of the repair patch and to a method for repairing a vehicle tire. The repair patch has a cover layer, a connecting layer for placement on a damaged spot of the elastomeric component, and at least one intermediate layer, which is arranged between the cover layer and the connecting layer. The repair patch has external dimensions such that a dimensional quotient which is at least formed by means of at least one size parameter of the damaged spot is within a predetermined value range and the number of intermediate layers depends on the at least one size parameter of the damaged spot.
PTL 3: EP 3495166 A1 discloses a vehicle tire having an outer surface, wherein the outer surface has an optoelectronically readable code which is formed by a relief from a matrix arrangement of a plurality of first surface regions having a depth profile and a plurality of second surface regions having a depth profile, wherein the first surface regions differ from the second surface regions in their depth profile such that they are designed to be optoelectronically distinguishable, and wherein the relief has a relief depth of a maximum of 0.5 mm. The task is to provide a vehicle tire with an optoelectronically readable code, whereby the vehicle tire has improved durability. This is achieved in that the outer surface having the optoelectronically readable code is a surface of a tread of the vehicle tire.
PTL 4: US 2006042366 A1 discloses a temperature indicator that includes at least one reactive substance and at least one dye substance. The at least one reactive substance has a threshold temperature and the at least one dye substance has at least one characteristic peak in its absorption or emission spectrum. When an excess temperature is reached in the tire, the at least one reactive substance is heated above the threshold temperature and reacts with the at least one dye substance so as to modify the at least one characteristic peak. A related pneumatic tire includes at least one such temperature indicator.

### SUMMARY

### (Technical Problem)

However, the technology in PTL 1 displays a pre-printed expiration date, which is not accurate enough to be used as an objective index of the degradation state of the tire and of the rubber members that constitute the tire.

In light of such circumstances, it is an aim of the present invention to provide a repair patch and a pre-cured treadthat have an indicator function enabling determination of the degradation state of a tire with high accuracy.

### (Solution to Problem)

A repair patch according to an embodiment of the present invention is a repair patch to be attached to a tire and includes an indicator portion having color components to be extracted by a degradation state determination apparatus that includes a degradation state model and a color change database for determining a degradation state of the tire, and a reinforcement portion to be bonded to the tire to reinforce the tire. The indicator portion includes a medium layer having printed thereon an identifier and at least one color pattern having a plurality of colors applied to different positions, the plurality of colors having different sensitivities to a degradation factor.

A pre-cured tread according to an embodiment of the present invention includes an indicator portion having color components to be extracted by a degradation state determination apparatus that includes a degradation state model and a color change database for determining a degradation state of a tire, and a main body configured by vulcanized tread rubber. The indicator portion includes a medium layer having printed thereon an identifier and at least one color pattern having a plurality of colors applied to different positions, the plurality of colors having different sensitivities to a degradation factor.

### (Advantageous Effect)

According to the present invention, a repair patch and a pre-cured tread that have an indicator function enabling determination of the degradation state of a tire with high accuracy can be provided.

### BRIEF DESCRIPTION OF THE DRAWINGS

In the accompanying drawings:
FIG. 1 is a diagram illustrating a configuration example of a degradation state determination apparatus;
FIG. 2 is a diagram illustrating a configuration example of a degradation state determination system that includes the degradation state determination apparatus in FIG. 1;
FIG. 3 is a diagram illustrating a configuration example of an indicator;
FIG. 4 is a flowchart illustrating an example of a first process included in a degradation state determination method executed by the degradation state determination apparatus in FIG. 1;
FIG. 5 is a diagram illustrating a configuration example of a color change database;
FIG. 6 is a flowchart illustrating an example of a second process included in the degradation state determination method executed by the degradation state determination apparatus in FIG. 1;
FIG. 7 is a diagram illustrating a configuration example of an indicator database;
FIG. 8 is a flowchart illustrating an example of a third process included in the degradation state determination method executed by the degradation state determination apparatus in FIG. 1;
FIG. 9 is a diagram of a repair patch according to an embodiment of the present invention;
FIG. 10 is a cross-sectional diagram of the repair patch according to an embodiment of the present invention;
FIG. 11 is a diagram illustrating how a hole in a tire is blocked by a repair patch;
FIG. 12 is a diagram of a pre-cured tread according to an embodiment of the present invention;
FIG. 13 is a cross-sectional diagram of the pre-cured tread according to an embodiment of the present invention;
FIG. 14 is a diagram of a tire not according to an embodiment of the present invention but useful for understanding the present invention; and
FIG. 15 is a cross-sectional diagram of the tire not according to an embodiment of the present invention but useful for understanding the present invention.

### DETAILED DESCRIPTION

### (First Embodiment)

An indicator is described below with reference to the drawings. Parts in the drawings that are the same or correspond are allotted the same reference signs. In the explanation of the present embodiment, a description of identical or equivalent portions is omitted or simplified as appropriate. The indicator is used for determination, by a degradation state determination apparatus, of the degradation state of a target object to which the indicator is attached. First, the degradation state determination apparatus is described.

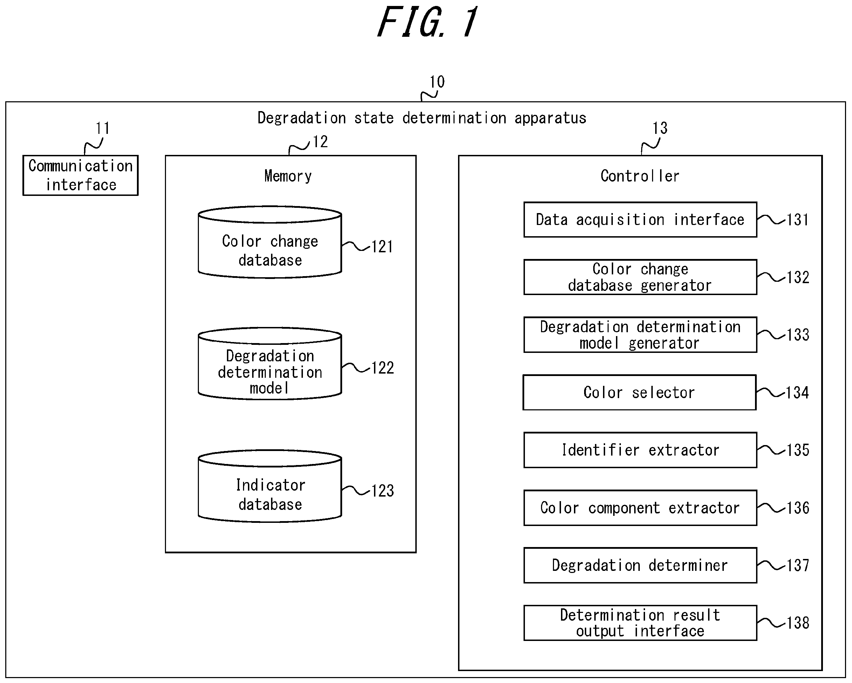

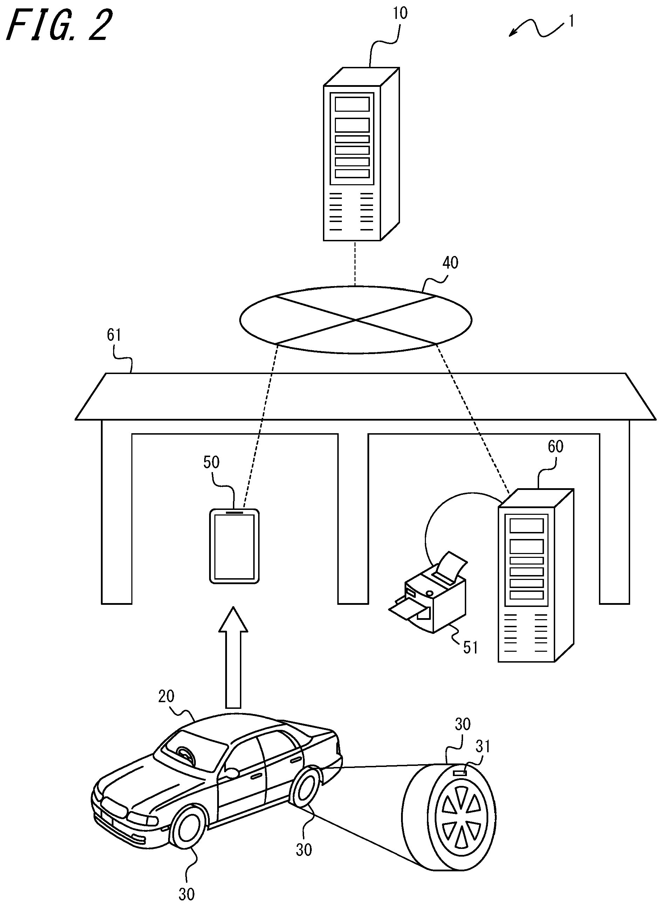

FIG. 1 is a diagram illustrating a configuration example of a degradation state determination apparatus 10. FIG. 2 is a diagram illustrating a configuration example of a degradation state determination system 1 that includes the degradation state determination apparatus 10 in FIG. 1. The degradation state determination apparatus 10 is an apparatus for determining the degradation state of a target object that is the target of determination of the degradation state based on a color of an indicator 31 attached to the target object. In the present embodiment, the target object is described as being a tire 30, but the target object is not limited to being the tire 30. The degradation state of the tire 30 determined by the degradation state determination apparatus 10 may, for example, be used to present the expected service life, a suitable vulcanization method, or the like to the user in the case of retreading the tire 30. Although the degradation of the tire 30 includes factors not apparent from the external appearance (such as hardening), the degradation can be determined with high accuracy by the degradation state determination apparatus 10. Retreading refers to the process of scraping off the tread rubber of the tire 30, affixing and vulcanizing new rubber, and then reusing the tire 30. A pre-cured tread (PCT) is sometimes used. The user is the user of a management facility 61 for the tire 30 where retreading and other operations are performed. In particular, the user is the driver or owner of the vehicle 20.

The degradation state determination apparatus 10 includes a communication interface 11, a memory 12, and a controller 13. The controller 13 includes a data acquisition interface 131, a color change database generator 132, a degradation determination model generator 133, a color selector 134, an identifier extractor 135, a color component extractor 136, a degradation determiner 137, and a determination result output interface 138. In terms of hardware configuration, the degradation state determination apparatus 10 may, for example, be a computer such as a server. Details of the constituent elements of the degradation state determination apparatus 10 are described below.

The degradation state determination apparatus 10, together with at least one of a terminal apparatus 50, a printing apparatus 51, and a server 60 connected by a network 40, may form a degradation state determination system 1. The network 40 is, for example, the Internet, but may be a Local Area Network (LAN).

The terminal apparatus 50 is a general purpose mobile terminal such as a smartphone or tablet terminal but is not limited to such a mobile terminal, as long as the terminal apparatus 50 includes an imaging function and a display function. The terminal apparatus 50 is used by an administrator or user of the management facility 61 when, for example, the degradation state of the tire 30 is determined. The terminal apparatus 50 may use the imaging function to capture an image of the indicator 31 attached to the tire 30 and transmit the resulting image to the degradation state determination apparatus 10 via the network 40. The imaging function is, for example, realized by a camera included in the terminal apparatus 50. The terminal apparatus 50 may also acquire the degradation state determination result from the degradation state determination apparatus 10 via the network 40 and use the display function to display the result to the user. The display function is, for example, realized by a display such as an LCD included in the terminal apparatus 50. Here, the terminal apparatus 50 may include a touch panel display integrated with a touch sensor that detects contact by the user and identifies the contact position.

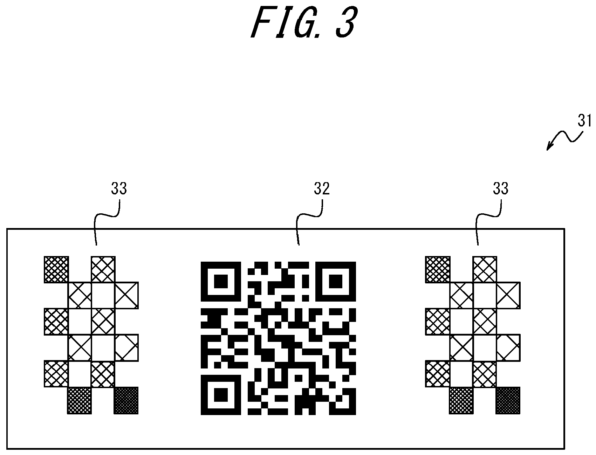

The printing apparatus 51 is, for example, a color inkjet printer but is not limited to a color inkjet printer, as long as the apparatus is capable of printing a plurality of color components. The printing apparatus 51 is used to print an identifier 32 and a color pattern 33 (see FIG. 3), described below, on a print medium such as paper or film (membranous resin), rubber, or the like when creating the indicator 31 to be attached to the tire 30. In the present embodiment, the printing apparatus 51 acquires print data from the degradation state determination apparatus 10 via the network 40 and the server 60 and performs printing to create the indicator 31. Here, the print data is indicator data including the identifier 32 and data on the color pattern 33.

The server 60 is, for example, a different computer than the degradation state determination apparatus 10. The server 60 is, for example, located in the management facility 61 and may manage data such as the retreading and repair history of the tires 30, in addition to relaying indicator data to the printing apparatus 51.

The management facility 61 is a facility for managing the tires 30 mounted on the vehicle 20. In the present embodiment, the tires 30 are repaired, replaced, retreaded, and the like at the management facility 61. Although the degradation state determination system 1 can execute the processing for degradation state determination regardless of the location of the vehicle 20, the processing for degradation state determination is described in the present embodiment as being performed while the vehicle 20 is visiting the management facility 61. The terminal apparatus 50, the printing apparatus 51, and the server 60 may be located inside the management facility 61 or at a separate location from the management facility 61.

An overview of the degradation state determination method performed by the degradation state determination apparatus 10 is now described. Generally, it is known that printed materials printed by color inkjet printers or the like fade as the ink ages. The degree of fading varies depending on the color of the ink, even under the same environmental conditions. The degree of fading also differs depending on degradation factors in the environment (such as heat or ultraviolet light), even for the same print material. In the present embodiment, at the time of replacement of the tire 30, for example, an indicator 31 is attached to the new tire 30 (see FIG. 2). The degradation state determination apparatus 10 later acquires an image of the indicator 31 and calculates the amount of a degradation factor in the environment in which the tire 30 was used from the change in color of the indicator 31. The degradation state determination apparatus 10 then determines the degradation state of the tire 30 based on the calculated amount of the degradation factor.

FIG. 3 is a diagram illustrating a configuration of the indicator 31 according to the present embodiment. The indicator 31 includes an identifier 32 and color patterns 33 printed on a print medium. The indicator 31 can be created inexpensively by printing with the printing apparatus 51. The identifier 32 is a two-dimensional code in the present embodiment but is not limited to a two-dimensional code, as long as the indicator 31 (or a repair patch 70, as described below) can be individually identified. The color pattern 33 is a pattern formed by applying a plurality of colors at different locations. The color pattern 33 is arranged in a checkerboard pattern in the present embodiment but is not limited to such an arrangement. The degradation state determination apparatus 10 determines the degradation state of the tire 30 based on the change in color of the color pattern 33. The selection of colors used in the color pattern 33 and other details are described below.

In the example in FIG. 2, the indicator 31 is provided on the sidewall of the tire 30, but the location where the indicator 31 is attached is not limited. As another example, the indicator 31 may be affixed to the surface of the inner liner of the tire 30 being retreaded. As yet another example, the indicator 31 may be attached to the bottom of a groove in the tread portion. A plurality of indicators 31 may be respectively attached to different locations on the tire 30. In this case, even if some of the indicators 31 fall off during travel by the vehicle 20 or become too dirty to be identified, the remaining indicators 31 can still be used to identify a change in color of the color pattern 33. Another advantage of providing a plurality of indicators 31 is that if a third party intentionally heats some of the indicators 31 to cause them to degrade, the remaining indicators 31 can be used to prevent index manipulation. Provision of a plurality of indicators 31 also enables independent estimation of the degree of degradation of each component in the tire 30. The robustness of the degradation state determination is thereby improved.

The degradation state determination system 1 is not limited to the configuration illustrated in FIG. 2. In the present embodiment, the printing apparatus 51 is connected to the network 40 via the server 60, but the printing apparatus 51 may be directly connectable to the network 40, for example. In such a case, the server 60 may be omitted, and the degradation state determination system 1 may be configured by the degradation state determination apparatus 10, the terminal apparatus 50, and the printing apparatus 51.

The constituent elements of the degradation state determination apparatus 10 are now described in detail. The communication interface 11 is configured to include one or more communication modules that connect to the network 40. The communication interface 11 may include a communication module corresponding to mobile communication standards, such as 4G (4^{th} Generation) and 5G (5^{th} Generation). The communication interface 11 may include a communication module corresponding to wired LAN standards (for example, 1000BASE-T). The communication interface 11 may include a communication module corresponding to wireless LAN standards (for example, IEEE802.11).

The memory 12 includes one or more memories. The memory can, for example, be a semiconductor memory, a magnetic memory, or an optical memory, but is not limited to these examples and can be any memory. The memory 12 is, for example, built into the degradation state determination apparatus 10, but the memory 12 can also be configured to be accessed externally by the degradation state determination apparatus 10 via any interface.

The memory 12 stores various data used in the various calculations performed by the controller 13. The memory 12 may also store the results and intermediate data of the various calculations performed by the controller 13.

In the present embodiment, the memory 12 includes a color change database 121, a degradation determination model 122, and an indicator database 123. The color change database 121 includes data indicating the relationship between the change in color of the indicator 31 and the amount of the degradation factor that causes the tire 30 to degrade. The degradation determination model 122 is a model for determining the degradation state of the tire 30 based on the amount of a degradation factor. The degradation determination model 122 may, for example, be generated by machine learning or the like and may be a mathematical model that takes the amount of the degradation factor as input and outputs a predicted service life. The indicator database 123 includes data that associates the identifier 32 of the indicator 31 with information on the color pattern 33. The information on the color pattern 33 includes the colors used, the color components that make up each color, and so on. The information on the color pattern 33 may also include the position of each color, the type of pattern for the color arrangement (such as a checkerboard pattern), and the like. The information on the color pattern 33 may also include the initial state of each color by gradation of each color component. Here, the initial state is the state of the indicator 31 at the time of creation.

The controller 13 includes one or more processors. The processors can, for example, be a general purpose processor or dedicated processor specialized for specific processing, but these examples are not limiting, and any processor may be used. The controller 13 controls the overall operations of the degradation state determination apparatus 10.

Here, the degradation state determination apparatus 10 may have the following software configuration. One or more programs used to control the operations of the degradation state determination apparatus 10 are stored in the memory 12. When read by the processor of the controller 13, the programs stored in the memory 12 cause the controller 13 to function as the data acquisition interface 131, the color change database generator 132, the degradation determination model generator 133, the color selector 134, the identifier extractor 135, the color component extractor 136, the degradation determiner 137, and the determination result output interface 138.

The data acquisition interface 131 acquires experimental data and images of the indicator 31, described below, via the network 40 and the communication interface 11.

The color change database generator 132 generates the color change database 121 and stores the generated color change database 121 in the memory 12. The color change database generator 132 can generate the color change database 121 by extracting information on colors and information on degradation factors from experimental data and generating data indicating the relationship between the change in color and the amount of a degradation factor.

The degradation determination model generator 133 generates the degradation determination model 122 and stores the generated degradation determination model 122 in the memory 12. The degradation determination model 122 is a model that takes an amount of a degradation factor as input and outputs an index of the degradation state of the tire 30. The index of the degradation state of the tire 30 is, for example, an index pertaining to durability, such as service life, or an index pertaining to driving performance, such as rolling resistance and wet grip, but these examples are not limiting. The degradation determination model generator 133 may acquire performance data, acquirable via the network 40 and the communication interface 11, indicating the actual degradation state of the tire 30 and may generate the degradation determination model 122 based on the performance data. The performance data may, for example, be data that is obtained from actual driving or testing of the vehicle 20 and indicates the degradation state of the tire 30 when a specific amount of a specific degradation factor is provided to the tire 30. As an example, the performance data may indicate the actual service life of the tire 30 when a new tire 30 of a specific type is exposed to an environment with a temperature of 20°C to 80°C and 20% to 80% oxygen for 10 to 10000 hours. The degradation determination model generator 133 may generate the degradation determination model 122 by machine learning using the performance data as training data.

The color selector 134 selects colors to be used for the indicator 31 so that the amount of the degradation factor can be identified in the degradation state determination performed by the degradation determiner 137. The color selector 134 then outputs the indicator data (print data) so that the color pattern 33 including the selected colors is printed by the printing apparatus 51.

The identifier extractor 135 extracts the identifier 32 from the image of the indicator 31 acquired by the data acquisition interface 131. The identifier 32 extracted by the identifier extractor 135 is used to identify the indicator 31 in the degradation state determination performed by the degradation determiner 137.

The color component extractor 136 extracts color components of the colors included in the color pattern 33 from the image of the indicator 31 acquired by the data acquisition interface 131. The color components extracted by the color component extractor 136 are used to identify the amount of the degradation factor in the degradation state determination performed by the degradation determiner 137. The color component extractor 136 may extract color component values in the RGB color space, HSV color space, and Lab color space at the position of each ink identified in the image and may execute a process to calculate an average value among color component values of the same color.

The degradation determiner 137 uses the degradation determination model 122 to determine the degradation state of the tire 30 based on the color change database 121 and the color components extracted from the image of the indicator 31 by the color component extractor 136. In detail, the degradation determiner 137 estimates (calculates) the amount of the degradation factor affected by the environment in which the tire 30 is used by comparing the color components extracted from the image of the indicator 31 with the data in the color change database 121. The degradation determiner 137 inputs the estimated amount of the degradation factor into the degradation determination model 122 and takes the outputted index of the degradation state of the tire 30 as the determination result.

In the present embodiment, the degradation determiner 137 calculates the color components extracted from the image of the indicator 31 as a color component change by comparing the color components with the initial state of the indicator 31. The degradation determiner 137 can obtain the initial state of the color components of the indicator 31 from the indicator database 123. The degradation determiner 137 estimates the amount of the degradation factor by comparing the color component change with the data in the color change database 121. As a result of the color component change being obtained by comparison with the initial state in the present embodiment, the change in color can be accurately determined even if there are individual differences (such as printing variations) at the time the indicator 31 is created. The degradation state of the target object can therefore be determined with even higher accuracy.

The determination result output interface 138 outputs the determination result of the degradation state determination performed by the degradation determiner 137. The determination result outputted by the determination result output interface 138 is displayed on a display or the like of the terminal apparatus 50. For example, in a case in which the server 60 is configured to be connected to another display, the determination result may be displayed on the display connected to the server 60. Based on the determination result, the manager of the management facility 61, for example, can present the user with the expected service life in the case of replacing, repairing, or retreading the tire 30, a suitable vulcanization method, or the like.

The degradation state determination method performed by the degradation state determination apparatus 10 is described below with reference to flowcharts and the like. The degradation state determination method includes a first process, a second process, and a third process. The first process is for generating the color change database 121 and the degradation determination model 122 and is executed before the second and third processes. The second process is for creating (printing by the printing apparatus 51) the indicator 31 to be attached to the tire 30 and is executed before the third process. The third process is for acquiring an image of the indicator 31 attached to the tire 30 and determining the degradation state of the tire 30.

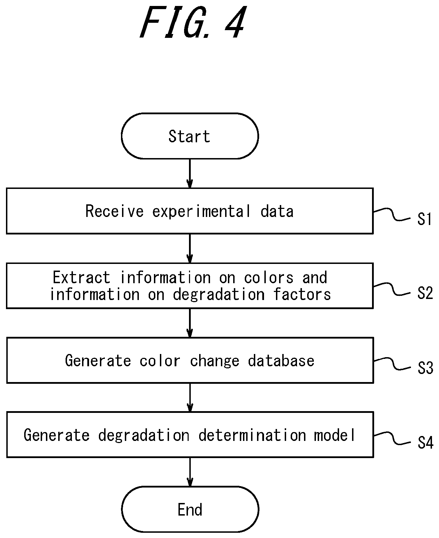

FIG. 4 is a flowchart illustrating an example of the first process included in the degradation state determination method.

The data acquisition interface 131 receives experimental data (step S1). The experimental data is data obtained by experimentally providing, to an experimental indicator created by the printing apparatus 51 in the same manner as the indicator 31, a degradation factor to cause the colors to fade. Similar to the color pattern 33 of the indicator 31, the experimental indicator has a pattern in which a plurality of colors is arranged. The experimental data includes information on the colors and information on the degradation factor. The experimental data may include a captured image of the experimental indicator and information on the degradation factor in the form of text information. The information on the degradation factor indicates the amount of the degradation factor provided experimentally. This may, for example, be information such as 20 hours of exposure to an environment with a temperature of 20°C and 20% oxygen.

The experimental data is transmitted to the degradation state determination apparatus 10 by, for example, the terminal apparatus 50. An application installed on the terminal apparatus 50 may associate the image of the experimental indicator with the information on the degradation factor and transmit the combination as the experimental data to the degradation state determination apparatus 10. Here, the terminal apparatus 50 may be at a different location (such as an experimental facility) than the management facility 61 and transmit the experimental data.

The color change database generator 132 extracts information on colors and information on degradation factors from the experimental data (step S2).

The color change database generator 132 generates data illustrating the relationship between the change in color and the amount of the degradation factor, and associates the data to generate the color change database 121 (step S3, color change database generation step).

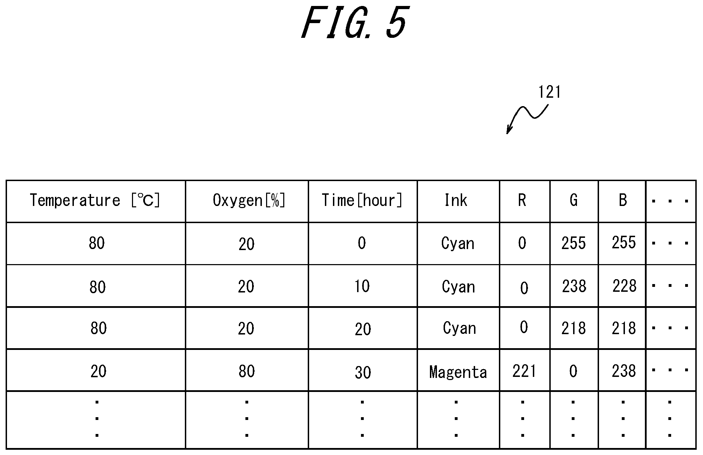

FIG. 5 is a diagram illustrating a configuration example of the color change database 121. In the example in FIG. 5, the color change database 121 is configured as a set of data that associates temperature (Temperature), oxygen (Oxygen), time exposed to conditions (Time), color or the ink used (Ink), and color components of the color used (R, G, B). The ink is, for example, indicated as cyan, magenta, yellow, or the like. The color components are indicated by a gradation from 0 to 255 for each of red (R), green (G), and blue (B).

In the example in FIG. 5, it is clear from the first and second columns of the color change database 121 that cyan, which had 255 G and 255 B as color components, fades to 238 G and 228 B when exposed to an environment with a temperature of 80°C and 20% oxygen for 10 hours. The color change database 121 thus indicates the relationship between changes in color and the amounts of degradation factors. When a change in color (fading) occurs in the color pattern 33 of the indicator 31, these relationships can be used to estimate the amount of the degradation factor that caused the fading by searching for data in the color change database 121 that indicates the same color component change.

The degradation determination model generator 133 generates the degradation determination model 122 (step S4). As described above, the degradation determination model 122 is a model that takes an amount of a degradation factor as input and outputs an index of the degradation state of the tire 30. Generation of the degradation determination model 122 in addition to the color change database 121 enables output of an index of the degradation state of the tire 30 based on the fading of the color pattern 33 of the indicator 31, using the estimated amount of the degradation factor as an intermediate parameter.

The items in the color change database 121 are not limited to the example in FIG. 5. For example, the degradation factors may include at least one of heat, oxygen, water, ultraviolet light, and ozone. By inclusion of such degradation factors that can be assumed in an outdoor environment, the accuracy of determining the degradation state of target objects used outdoors, including tires 30, can be improved.

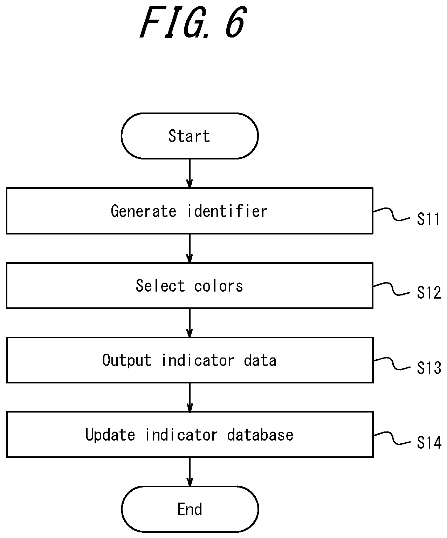

FIG. 6 is a flowchart illustrating an example of the second process included in the degradation state determination method. The second process is executed after the first process. The second process is, for example, executed when the tire 30 of the vehicle 20 is replaced with a new tire 30 at the management facility 61.

The color selector 134 generates an identifier 32 to be printed on the indicator 31 (step S11). The identifier 32 is used to associate the indicator 31 and the information on the color pattern 33 in the indicator database 123. The identifier 32 enables identification of each of the indicators 31, thus enabling the initial state to be recognized and individually managed. The color selector 134 may, for example, use a UUID (Universally Unique Identifier).

The color selector 134 selects the colors to be used for the indicator 31 so that the amount of the degradation factor can be identified (step S12, color selection step). As described above, the degree of fading depends on the degradation factor in the environment and the color of the ink. The color selector 134 may first select a color that varies significantly (color with high sensitivity) depending on the degradation factor assumed in the environment in which the tire 30 is used. For example, the color selector 134 may select a color (such as magenta) that fades significantly due to high temperature (such as 80°C) when high temperature is assumed as a degradation factor. The color selector 134 may further select a color for contrast. For example, the color selector 134 may select a color (such as yellow) that fades due to high temperature only under aerobic conditions as a contrast color. In a case in which no fading in yellow is observed whereas magenta is significantly faded during the degradation state determination, for example, the degradation factor can more accurately be identified as high temperature rather than oxygen. Here, the color selector 134 refers to the color change database 121 in selecting colors. The color selector 134 also specifies the color components of the selected colors based on the data in the color change database 121 so that the amount of the degradation factor can be accurately identified during the degradation state determination. The color selector 134 specifies, for example, magenta with R of 255 and B of 255. The color selector 134 also determines the arrangement of each color in the color pattern 33. The position at which each color is arranged may be managed by coordinates.

The color selector 134 selects, for inclusion in the indicator data, colors with color components that are less likely to fade for the sake of the control correction described below.

In the present embodiment, the color selector 134 selects a plurality of colors that can identify the same degradation factor and generates indicator data so that each of the colors that can identify the same degradation factor is applied to a different position in the indicator 31. By the color pattern 33 being configured in this way, even if a portion of the indicator 31 becomes unidentifiable due to dirt or the like after being attached to the tire 30, the degradation factor can still be identified from the remaining colors. As in the example in FIG. 3, the color selector 134 may generate the indicator data so that a plurality of color patterns 33 are in different positions.

The color selector 134 outputs the indicator data including the identifier 32 and the data on the color pattern 33 (step S13). The printing apparatus 51 acquires the indicator data from the degradation state determination apparatus 10 as print data via the network 40 and the server 60 and creates the indicator 31 by printing. The created indicator 31 is attached to the tire 30. In the present embodiment, an image of the indicator 31 created by the terminal apparatus 50 is captured, and the image is transmitted to the degradation state determination apparatus 10 as an indication of the initial state of the indicator 31.

The color selector 134 updates the indicator database 123 (step S14). In other words, information on the indicator data outputted by the color selector 134 is added to the indicator database 123. The information about the created indicator 31 is managed by the indicator database 123.

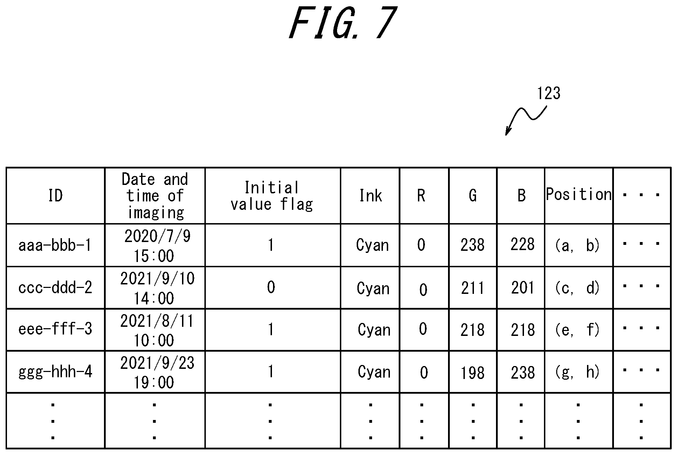

FIG. 7 is a diagram illustrating a configuration example of the indicator database 123. In the example in FIG. 7, the indicator database 123 is configured by a group of data in which the identifier 32 (ID), date and time of imaging, initial value flag, ink (Ink), color components (R, G, B), and color positions are associated. For a newly created indicator 31, information on the identifier 32 and color positions is extracted from the indicator data, and the data is added with the initial value flag set to "1". As described above, an image of the indicator 31 created by the terminal apparatus 50 is transmitted to the degradation state determination apparatus 10. The date and time of imaging, ink, and color components of this image are extracted and added to data on the newly created indicator 31. Here, the extraction of ink and color components may be performed by the color component extractor 136, and the color selector 134 may acquire the execution result of the color component extractor 136. In the indicator database 123, data with an initial value flag of "1" includes the initial state of the color of the indicator 31 having that identifier 32. Data with an initial value flag of "0" includes the color state after the indicator 31 having that identifier 32 is attached to the tire 30, and this data is added to the indicator database 123 by the third process.

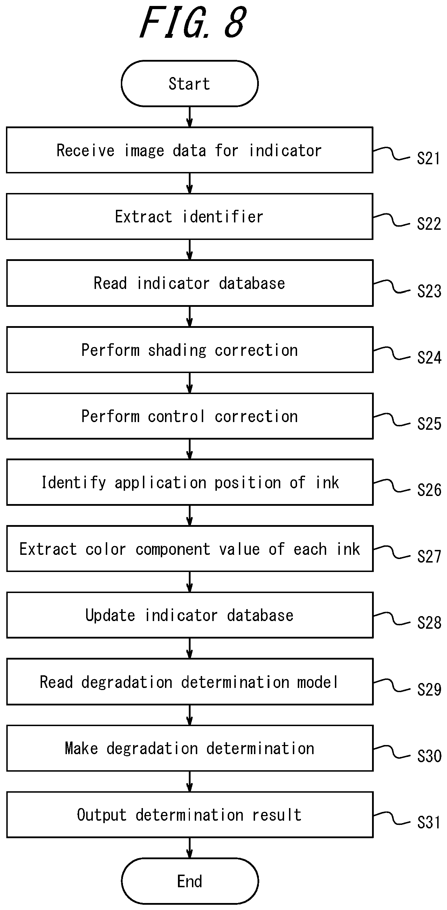

FIG. 8 is a flowchart illustrating an example of the third process included in the degradation state determination method. The third process is executed after the second process. The third process is, for example, executed at the timing when maintenance of the tires 30 is performed at the management facility 61. The third process may therefore be executed multiple times.

The data acquisition interface 131 receives the image data for the indicator 31 (step S21).

The identifier extractor 135 extracts the identifier 32 from the image data acquired by the data acquisition interface 131 (step S22).

The degradation determiner 137 identifies the indicator 31 based on the identifier 32 extracted by the identifier extractor 135. The degradation determiner 137 reads the indicator database 123 (step S23) and acquires data such as the initial state of the color of the identified indicator 31.

The color component extractor 136 performs shading correction (step S24) on the image data acquired by the data acquisition interface 131. Shading correction corrects for differences in light intensity within the color pattern 33 caused by the imaging environment.

The color component extractor 136 performs control correction (step S25). The control correction is performed to accurately measure the change from the initial state of the color of the indicator 31. The color component extractor 136 extracts a color component in the color pattern 33 that is less likely to fade (such as the G component of cyan) and adjusts the gradation to the initial state. This process allows the light intensity to be matched to the image of the indicator 31 at the time of creation.

The color component extractor 136 identifies the application positions of all inks (colors) that the color pattern 33 has based on the data in the indicator database 123 (step S26). For example, the positions are identified for cyan, magenta, yellow, and at least some mixture of these colors included in the color pattern 33.

The color component extractor 136 extracts the color component value of each ink whose position has been identified (step S27, color component extraction step). For example, in a case in which a plurality of color patterns 33 is included as in the example in FIG. 3, an average value may be used as the color component value for each ink. In a case in which a portion is dirty, the color component value of each ink may be extracted using only the color pattern 33 in the region that is not dirty. Although degradation determination may be performed using the extracted color component values of each ink themselves, degradation determination is performed in the present embodiment using the difference from the initial state. The color component extractor 136 therefore calculates the change from the initial state of the color component value of each ink using the data in the indicator database 123.

Here, the color component extractor 136 updates the indicator database 123 in the same manner as in step S14 of the second process (step S28). The configuration of the data added to the indicator database 123 is the same as in step S14 of the second process, except that the initial value flag is "0".

The degradation determiner 137 reads the color change database 121. The degradation determiner 137 also reads the degradation determination model 122 (step S29).

The degradation determiner 137 uses the degradation determination model 122 to make a degradation determination for the tire 30 based on the color change database 121 and the color components extracted from the image (step S30, degradation determination step).

First, the degradation determiner 137 extracts data corresponding to the change from the initial state of the color component values from the color change database 121 to estimate the amount of the degradation factor that the tire 30 has received from the environment. For example, in a case in which cyan, which had 253 for G and 253 for B as color components in the initial state, has faded (changed) to 236 for G and 226 for B, the degradation determiner 137 calculates 17 for G and 27 for B as the color component change. The degradation determiner 137 may extract data corresponding to such a color component change based on the color change database 121 to estimate that the amount of the degradation factor is "10 hours of exposure to an environment with a temperature of 80°C and 20% oxygen". The degradation determiner 137 may perform the estimation with known methods, such as regression analysis on the data constituting the color change database 121.

The degradation determiner 137 may also calculate the color component change for a plurality of color components that exhibit different changes for one degradation factor and may identify the amount of the degradation factor from the plurality of color component changes. For example, assume that the color change database 121 contains data indicating that the R of magenta changes from 255 to 220 when "exposed for 10 hours to an environment with a temperature of 80°C and 20% oxygen". By calculating and comparing the change in R of magenta, for example, in addition to cyan in the above example, the degradation determiner 137 can increase the accuracy of estimating the amount of the degradation factor and can consequently determine the degradation state of the tire 30 with higher accuracy.

The degradation determiner 137 inputs the amount of the degradation factor into the degradation determination model 122 to calculate an index of the degradation state of the tire 30. The degradation determiner 137 may calculate a plurality of indices of the degradation state of the tire 30, including the service life.

The determination result output interface 138 outputs the determination result by the degradation determiner 137 (step S31). The administrator of the management facility 61 may propose a method or the like for replacing, repairing, or retreading the tire 30 to the user based on the index of the degradation state of the tire 30 indicated as the determination result.

As described above, the indicator 31 enables determination of the degradation state of a target object with high accuracy through the above configuration.

### (Second Embodiment)

In the first embodiment, the indicator 31 is created at the time of replacement of the tire 30 and is immediately attached to the new tire 30. The indicator 31 may, however, be attached to the target object after some time has elapsed since creation of the indicator 31. The indicator 31 may also be attached during the manufacturing process of the target object. In other words, the target object may be manufactured with the indicator 31 attached thereto. During distribution or the like of such a target object, an image of the initial state of the indicator 31 and an image at the time of determination of the degradation state can be uploaded to the degradation state determination apparatus 10 using the terminal apparatus 50, for example. The terminal apparatus 50 in this case may be an apparatus owned by the user, or the seller or manufacturer of the target object, who wishes to know the degradation state. The determination result by the degradation state determination apparatus 10 may be displayed on the terminal apparatus 50 via the network 40. An application installed on the terminal apparatus 50 may execute operations from the uploading of the image of the indicator 31 through the displaying of the determination result by the degradation state determination apparatus 10.

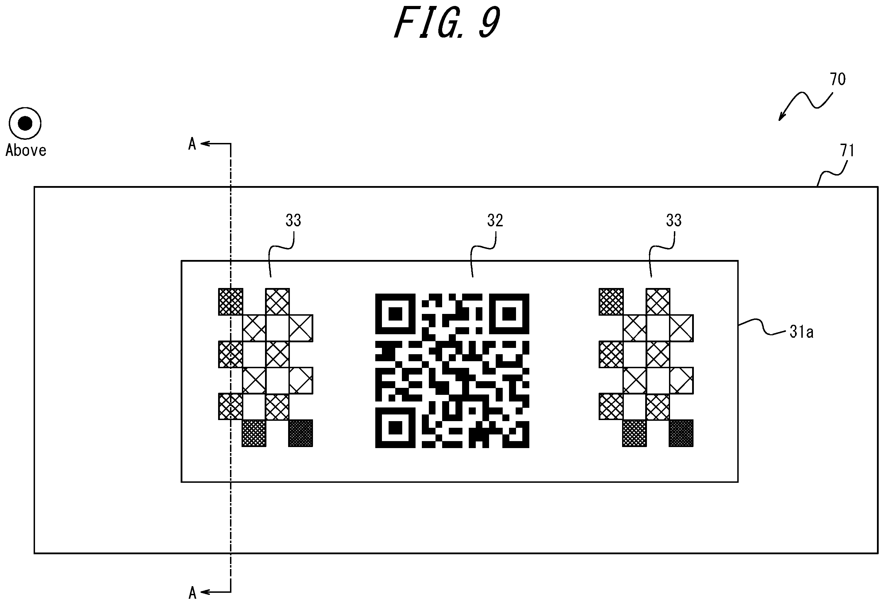

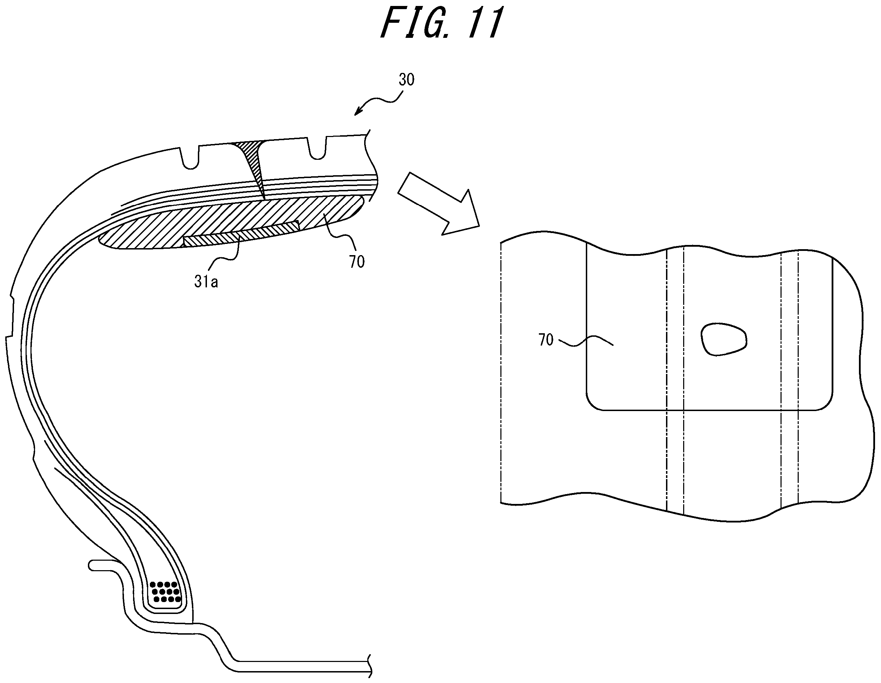

Here, in a case in which the tire 30 is punctured, a repair patch 70 (see FIG. 9) may be used to block the hole and repair the tire 30. FIG. 11 is a diagram illustrating how a hole in the tire 30 is blocked by the repair patch 70. The repair patch 70 according to the present embodiment has the function of the indicator 31 and, as in the first embodiment, enables determination of the degradation state of the tire 30 after the repair patch 70 is attached. To avoid duplicate explanation, a configuration different from the first embodiment is described below.

FIG. 9 is a diagram illustrating a configuration of the repair patch 70 according to the present embodiment. The repair patch 70 includes an indicator portion 31a and a reinforcement portion 71. The indicator portion 31a functions in the same manner as the indicator 31 in the first embodiment and includes an identifier 32 and a color pattern 33. The reinforcement portion 71 is bonded to and reinforces the tire 30. The reinforcement portion 71 may be of any known configuration.

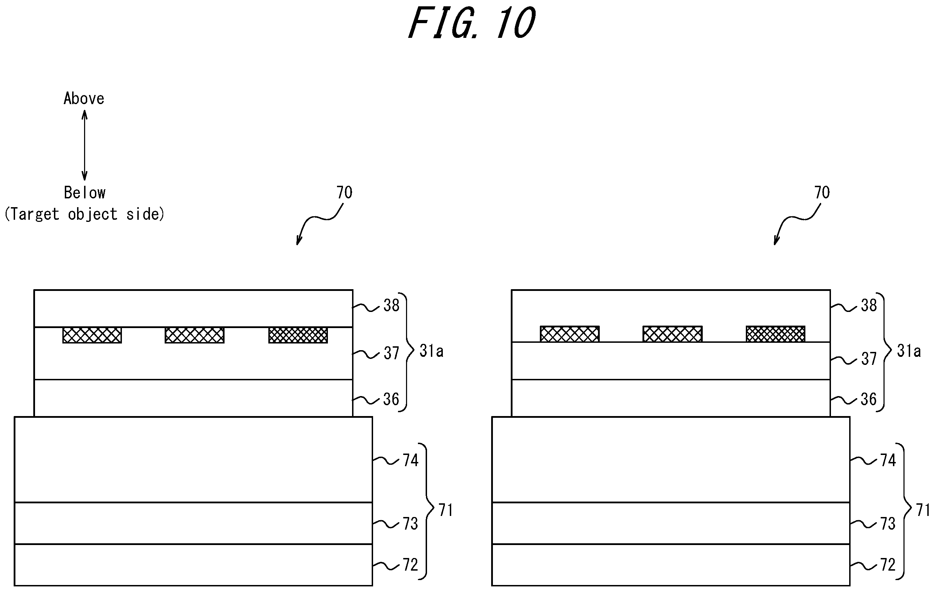

FIG. 10 is a cross-sectional diagram of the repair patch 70 in FIG. 9 and illustrates the layer structure of the repair patch 70 at the A-A cross-section in FIG. 9. In the present embodiment, the indicator portion 31a of the repair patch 70 includes a penetration prevention layer 36, a medium layer 37, and a protective layer 38. The reinforcement portion 71 of the repair patch 70 includes an adhesion layer 72, an air permeation prevention layer 73, and a reinforcement layer 74. The bottom surface of the adhesion layer 72 illustrated in FIG. 10 is an adhesion surface that adheres to the tire 30. As illustrated in FIG. 10, in the lamination direction, the positional relationships between layers can be described by designating the direction away from the target object (tire 30) as "above", and the direction closer to the target object as "below". FIG. 9 corresponds to a plan view of the repair patch 70 looking downward.

The medium layer 37 has printed thereon an identifier 32 and color patterns 33 having a plurality of colors applied to different positions. The colors have different sensitivities to a degradation factor. The left side of FIG. 10 illustrates the case in which the printing apparatus 51 uses dye ink. The dye ink penetrates into and is fixed in the medium layer 37. The right side of FIG. 10 illustrates the case in which the printing apparatus 51 uses pigment ink. The pigment ink is fixed on the surface of the medium layer 37. The printing apparatus 51 may use dye ink, pigment ink, or both. The medium layer 37 may be paper, film, rubber, or the like.

The protective layer 38 is provided on the side of the medium layer 37 away from the tire 30, i.e. above the medium layer 37, and inhibits transmission of at least ultraviolet light. The protective layer 38 has the function of adjusting the amount of ultraviolet light so that excessive fading is not caused by ultraviolet light, which is one degradation factor. The protective layer 38 may also have the function of reliably separating a plurality of colors by filling the space between adjacent colors in the color pattern 33. The function of the protective layer 38 to separate colors is particularly useful in a case in which pigment ink is used. The protective layer 38 may be a transparent acrylic resin or the like. The repair patch 70 may be configured without the protective layer 38.

The term "above" in the phrase "above the medium layer 37" includes not only being directly above the medium layer 37, but also the case of the additional presence of another layer between the medium layer 37 and that which is above the medium layer 37. For example, the protective layer 38 may be directly above the medium layer 37, or another layer may be present directly above the medium layer 37, with the protective layer 38 being above that other layer. The term "above" retains the same meaning in the case of expressing the relationship between other layers. The term "below" similarly includes the case of the additional presence of other layers.

The penetration prevention layer 36 is provided between the medium layer 37 and the reinforcement portion 71 and prevents penetration of ink. The penetration prevention layer 36 can suppress functional decline of an age resistor blended in the rubber for the tire 30. The penetration prevention layer 36 may, for example, be configured to include a urethane resin that absorbs ink. The repair patch 70 may be configured without the penetration prevention layer 36.

The adhesion layer 72 is provided on the side of the medium layer 37 closer to the tire 30, i.e., below the medium layer 37, and includes an adhesion surface that adheres to the tire 30. An adhesive may be applied to the adhesion surface. The adhesive may be a material such as epoxy resin, silicone resin, or urethane resin, but is not limited to these examples. The presence of the adhesion layer 72, which can adhere via the adhesive, enables the repair patch 70 to adhere to the tire 30 without heat treatment and thereby does not cause thermal aging of the indicator portion 31a during adhesion. The repair patch 70 may be configured without the adhesion layer 72.

The air permeation prevention layer 73 is provided between the medium layer 37 and the adhesion layer 72 and prevents air leakage from the tire 30. In the present embodiment, the air permeation prevention layer 73 is provided directly above the adhesion layer 72. The air permeation prevention layer 73 may, for example, be butyl rubber or the like.

The reinforcement layer 74 is provided between the medium layer 37 and the adhesion layer 72 and reinforces the punctured portion of the tire 30. In the present embodiment, the air permeation prevention layer 73 is provided directly below the indicator portion 31a and is configured as a textile member covered by a rubber member. The textile member may, for example, be polyester, nylon, or the like. The rubber member may, for example, be butyl rubber or the like. Here, the repair patch 70 may be configured to include only one of the air permeation prevention layer 73 and the reinforcement layer 74.

As described above, the repair patch 70 according to the present embodiment has an indicator function that, as in the first embodiment, enables determination of the degradation state of the tire with high accuracy.

### (Third Embodiment)

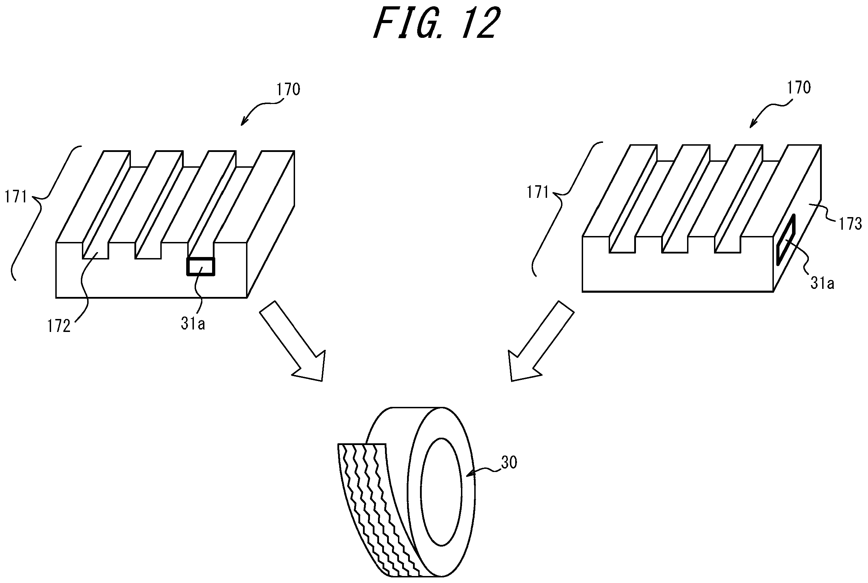

Here, a pre-cured tread 170 (see FIG. 12) may be used in retreading the tire 30. The pre-cured tread 170 includes a vulcanized tread rubber main body and is attached to a base tire during retreading after the tread rubber has been scraped off the base tire. The pre-cured tread 170 according to the present embodiment has the function of the indicator 31 and, as in the first embodiment, enables determination of the degradation state of the tire 30 after the pre-cured tread 170 is attached. The degradation state of the pre-cured tread 170 itself can also be determined. To avoid duplicate explanation, a configuration different from the first embodiment is described below.

FIG. 12 is a diagram illustrating a configuration of the pre-cured tread 170 according to the present embodiment. The pre-cured tread 170 includes an indicator portion 31a and a main body 171. The indicator portion 31a functions in the same manner as the indicator 31 in the first embodiment and includes an identifier 32 and color patterns 33. The main body 171 is adhered to the base tire of the tire 30 by adhesive or the like. The main body 171 may be of any known configuration. The pre-cured tread 170 may be manufactured by manufacturing the main body 171 by a known method and performing a process in which the indicator portion 31a is attached to the main body 171 by adhesion using an adhesive or by thermal adhesion. The adhesive may be a material such as epoxy resin, silicone resin, or urethane resin, but is not limited to these examples.

As illustrated in FIG. 12, the indicator portion 31a may be at the bottom of a groove 172 or on a side surface 173 of the tread portion. The indicator portion 31a may also be at another location other than the bottom of the groove 172 or the side surface 173. A plurality of indicator portions 31a may be provided and arranged at different locations so that the degradation state can be determined even if some of the indicator portions 31a are dirty.

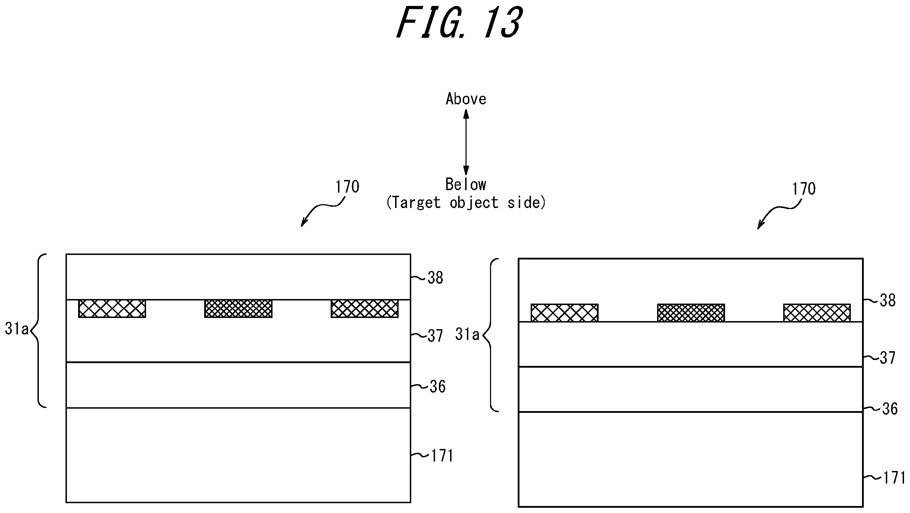

FIG. 13 is a cross-sectional diagram of the pre-cured tread 170 in FIG. 12 and illustrates the layer structure of a portion of the color pattern 33, such as the A-A cross-section in FIG. 9. In the present embodiment, the indicator portion 31a of the pre-cured tread 170 includes a penetration prevention layer 36, a medium layer 37, and a protective layer 38. The main body 171 of the pre-cured tread 170 is configured by vulcanized tread rubber and can, for example, be the bottom of the groove 172 or the side surface 173. The bottom surface of the main body 171 illustrated in FIG. 13 is an adhesion surface that adheres to the tire 30, which is a base tire. As illustrated in FIG. 13, in the lamination direction, the positional relationships between layers can be described by designating the direction away from the target object (tire 30) as "above", and the direction closer to the target object as "below".

The medium layer 37 has printed thereon an identifier 32 and color patterns 33 having a plurality of colors applied to different positions. The colors have different sensitivities to a degradation factor. The left side of FIG. 13 illustrates the case in which the printing apparatus 51 uses dye ink. The dye ink penetrates into and is fixed in the medium layer 37. The right side of FIG. 13 illustrates the case in which the printing apparatus 51 uses pigment ink. The pigment ink is fixed on the surface of the medium layer 37. The printing apparatus 51 may use dye ink, pigment ink, or both. The medium layer 37 may be paper, film, rubber, or the like.

The protective layer 38 is provided on the side of the medium layer 37 away from the tire 30, i.e. above the medium layer 37, and inhibits transmission of at least ultraviolet light. The protective layer 38 has the function of adjusting the amount of ultraviolet light so that excessive fading is not caused by ultraviolet light, which is one degradation factor. The protective layer 38 may also have the function of reliably separating a plurality of colors by filling the space between adjacent colors in the color pattern 33. The function of the protective layer 38 to separate colors is particularly useful in a case in which pigment ink is used. The protective layer 38 may be a transparent acrylic resin or the like. The indicator portion 31a may be configured without the protective layer 38.

The term "above" in the phrase "above the medium layer 37" includes not only being directly above the medium layer 37, but also the case of the additional presence of another layer between the medium layer 37 and that which is above the medium layer 37. For example, the protective layer 38 may be directly above the medium layer 37, or another layer may be present directly above the medium layer 37, with the protective layer 38 being above that other layer.

The penetration prevention layer 36 is provided between the medium layer 37 and the main body 171 and prevents penetration of ink. The penetration prevention layer 36 can suppress functional decline of an age resistor blended in the rubber for the tire 30. The penetration prevention layer 36 may, for example, be configured to include a urethane resin that absorbs ink. The indicator portion 31a may be configured without the penetration prevention layer 36.

As described above, the pre-cured tread 170 according to the present embodiment has an indicator function that, as in the first embodiment, enables determination of the degradation state of the tire 30 with high accuracy.

### (Fourth Embodiment)

Here, the target object to be manufactured with the indicator 31 attached thereto may be a replacement tire 30 or a tire 30 that is mounted on a vehicle 20 from the start. The tire 30 according to the present embodiment, not according to the invention but useful for understanding the invention, has the function of the indicator 31 and, as in the first embodiment, enables determination of the degradation state of the tire 30. To avoid duplicate explanation, a configuration different from the first embodiment is described below.

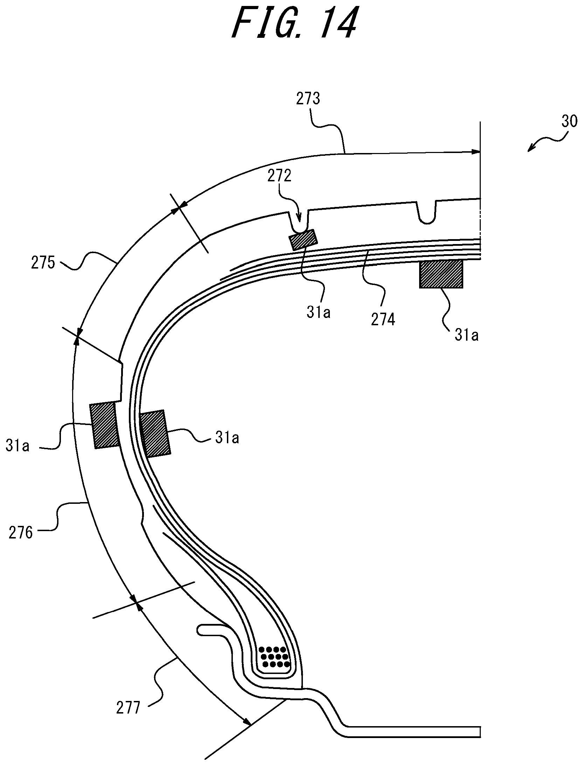

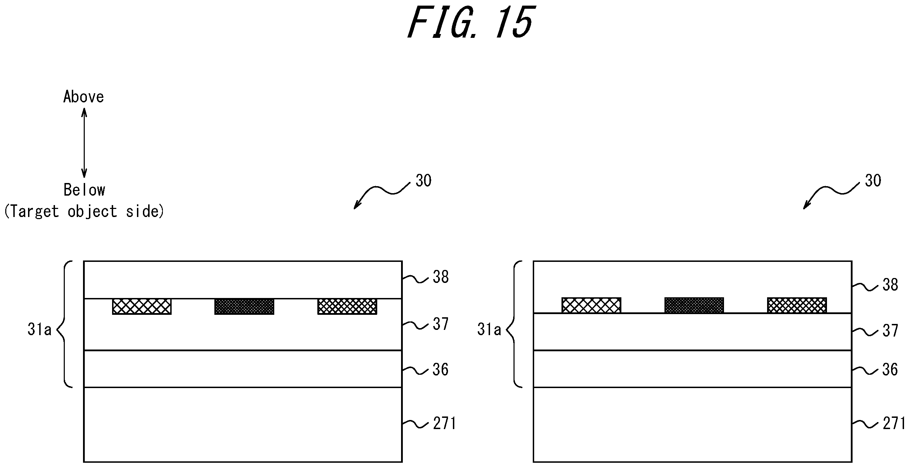

FIG. 14 is a diagram illustrating a configuration of the tire 30 according to the present embodiment. The tire 30 includes an indicator portion 31a and a main body 271 (see FIG. 15). The indicator portion 31a functions in the same manner as the indicator 31 in the first embodiment and includes an identifier 32 and color patterns 33. The main body 271 is a portion of the main body of the tire 30, which is formed by a rubber layer, a belt 274, a carcass, and the like. The indicator portion 31a is attached to the main body 271. The main body 271 may be of any known configuration. The tire 30 may be manufactured by manufacturing the main body 271 by a known method and performing a process in which the indicator portion 31a is attached to the main body 271 by adhesion using an adhesive or by thermal adhesion. The indicator portion 31a may be attached during the molding process prior to vulcanization, since the post-vulcanization condition may be utilized as the initial value. The adhesive may be a material such as epoxy resin, silicone resin, or urethane resin, but is not limited to these examples.

As illustrated in FIG. 14, the indicator portion 31a may be at the bottom of a groove 272 of the tread portion 273. The indicator portion 31a may be attached to the carcass at the inner portion of the belt 274. The indicator portion 31a may be on the outer portion or the inner portion of a sidewall portion 276 sandwiched between a shoulder portion 275 and a bead portion 277. A plurality of indicator portions 31a may be provided at least at two or more of these locations. Provision of a plurality of indicator portions 31a at different locations enables determination of the degradation state even if some of the indicator portions 31a are dirty.

FIG. 15 is a cross-sectional diagram of the indicator portion 31a of the tire 30 in FIG. 14 and illustrates the layer structure of a portion of the color pattern 33, such as the A-A cross-section in FIG. 9. In the present embodiment, the indicator portion 31a of the tire 30 includes a penetration prevention layer 36, a medium layer 37, and a protective layer 38. The main body 271 of the tire 30 can, for example, be the bottom of the groove 272. The bottom surface of the penetration prevention layer 36 illustrated in FIG. 15 can be an adhesion surface that adheres to the main body 271 of the tire 30. As illustrated in FIG. 15, in the lamination direction, the positional relationships between layers can be described by designating the direction away from the target object (the main body 271 of the tire 30 in the present embodiment) as "above", and the direction closer to the target object as "below".

The medium layer 37 has printed thereon an identifier 32 and color patterns 33 having a plurality of colors applied to different positions. The colors have different sensitivities to a degradation factor. The left side of FIG. 15 illustrates the case in which the printing apparatus 51 uses dye ink. The dye ink penetrates into and is fixed in the medium layer 37. The right side of FIG. 15 illustrates the case in which the printing apparatus 51 uses pigment ink. The pigment ink is fixed on the surface of the medium layer 37. The printing apparatus 51 may use dye ink, pigment ink, or both. The medium layer 37 may be paper, film, rubber, or the like.

The protective layer 38 is provided on the side of the medium layer 37 away from the main body 271, i.e. above the medium layer 37, and inhibits transmission of at least ultraviolet light. The protective layer 38 has the function of adjusting the amount of ultraviolet light so that excessive fading is not caused by ultraviolet light, which is one degradation factor. The protective layer 38 may also have the function of reliably separating a plurality of colors by filling the space between adjacent colors in the color pattern 33. The function of the protective layer 38 to separate colors is particularly useful in a case in which pigment ink is used. The protective layer 38 may be a transparent acrylic resin or the like. The indicator portion 31a may be configured without the protective layer 38.

The term "above" in the phrase "above the medium layer 37" includes not only being directly above the medium layer 37, but also the case of the additional presence of another layer between the medium layer 37 and that which is above the medium layer 37. For example, the protective layer 38 may be directly above the medium layer 37, or another layer may be present directly above the medium layer 37, with the protective layer 38 being above that other layer.

The penetration prevention layer 36 is provided between the medium layer 37 and the main body 271 and prevents penetration of ink. The penetration prevention layer 36 can suppress functional decline of an age resistor blended in the rubber for the tire 30. The penetration prevention layer 36 may, for example, be configured to include a urethane resin that absorbs ink. The indicator portion 31a may be configured without the penetration prevention layer 36.

As described above, the tire 30 according to the present embodiment has an indicator function that, as in the first embodiment, enables determination of the degradation state of the tire with high accuracy.

Although embodiments of the present invention have been described based on the drawings and examples, it is to be noted that various changes or modifications will be apparent to those skilled in the art based on the present invention. Therefore, such changes or modifications are to be understood as included within the scope of the present invention. For example, the functions and the like included in the components, steps, and the like may be reordered in any logically consistent way, and components, steps, and the like may be combined into one or divided. An embodiment of the present invention can also be realized as a program to be executed by a processor provided in an apparatus or as a storage medium with a program recorded thereon. Such embodiments are also to be understood as included within the scope of the present invention.

The image of the indicator 31 used to extract color components in the color component extraction step of the above embodiment is not limited to an image captured by irradiating visible light. For example, the image data for the indicator 31 may be an image captured by irradiating non-visible light such as ultraviolet (UV) light. In an image captured by irradiating non-visible light, the color change (sensitivity) to a specific degradation factor may be greater than in the case of irradiating visible light. Therefore, an image captured by irradiating with non-visible light may be used in cases in which the effect of a specific degradation factor is to be examined in detail.

### REFERENCE SIGNS LIST

- 1: Degradation state determination system
- 10: Degradation state determination apparatus
- 11: Communication interface
- 12: Memory
- 13: Controller
- 20: Vehicle
- 30: Tire
- 31: Indicator
- 31a: Indicator portion
- 32: Identifier
- 33: Color pattern
- 35: Adhesion layer
- 36: Penetration prevention layer
- 37: Medium layer
- 38: Protective layer
- 40: Network
- 50: Terminal apparatus
- 51: Printing apparatus
- 60: Server
- 61: Management facility
- 70: Repair patch
- 71: Reinforcement portion
- 72: Adhesion layer
- 73: Air permeation prevention layer
- 74: Reinforcement layer
- 121: Color change database
- 122: Degradation determination model
- 123: Indicator database
- 131: Data acquisition interface
- 132: Color change database generator
- 133: Degradation determination model generator
- 134: Color selector
- 135: Identifier extractor
- 136: Color component extractor
- 137: Degradation determiner
- 138: Determination result output interface
- 170: Pre-cured tread
- 171: Main body
- 172: Groove
- 173: Side surface
- 271: Main body
- 272: Groove
- 273: Tread portion
- 274: Belt
- 275: Shoulder portion
- 276: Sidewall portion
- 277: Bead portion

## Claims

1. A repair patch (70) to be attached to a tire (30), the repair patch (70) comprising:
a reinforcement portion (71) to be bonded to the tire (30) to reinforce the tire (30),
**characterized in that**
the repair patch further comprises:
an indicator portion having color components to be extracted by a degradation state determination apparatus that includes a degradation state model and a color change database for determining a degradation state of the tire, wherein
the indicator portion (31a) comprises a medium layer (37) having printed thereon an identifier (32) and at least one color pattern (33) having a plurality of colors applied to different positions, the plurality of colors having different sensitivities to a degradation factor.

2. The repair patch (70) according to claim 1, wherein the indicator portion (31a) further comprises a protective layer (38) provided on a side of the medium layer (37) away from the tire (30) and configured to inhibit transmission of at least ultraviolet light.

3. The repair patch (70) according to claim 1 or 2, wherein the reinforcement portion (71) comprises an adhesion layer (72) that is provided on a side of the medium layer (37) closer to the tire (30) and is to be adhered to the tire (30) by an adhesive.

4. The repair patch (70) according to any one of claims 1 to 3, wherein the indicator portion (31a) further comprises a penetration prevention layer (36) provided between the medium layer (37) and the reinforcement portion (71) and configured to prevent penetration of ink.

5. The repair patch (70) according to any one of claims 1 to 4, wherein the color pattern (33) is configured to include a plurality of color components that exhibit different changes for one degradation factor.

6. The repair patch (70) according to any one of claims 1 to 5, wherein the degradation factor includes at least one of heat, oxygen, water, ultraviolet light, and ozone.

7. The repair patch (70) according to any one of claims 1 to 6, wherein the at least one color pattern (33) comprises a plurality of color patterns printed on the medium layer (37).

8. A pre-cured tread (170) comprising:
an indicator portion (31a),
**characterized in that**
the pre-cured tread (170) further comprises:
a main body (171) configured by vulcanized tread rubber,
wherein the indicator portion (31a) has
color components to be extracted by a degradation state determination apparatus (10) that includes a degradation state model and a color change database (121) for determining a degradation state of a tire (30); and
wherein
the indicator portion (31a) comprises a medium layer (37) having printed thereon an identifier (32) and at least one color pattern (33) having a plurality of colors applied to different positions, the plurality of colors having different sensitivities to a degradation factor.

9. The pre-cured tread (170) according to claim 8, wherein the indicator portion (31a) further comprises a protective layer (38) provided on a side of the medium layer (37) away from the tire (30) and configured to inhibit transmission of at least ultraviolet light.

10. The pre-cured tread (170) according to claim 8 or 9, wherein the indicator portion (31a) further comprises a penetration prevention layer (36) provided between the medium layer (37) and the main body (171) and configured to prevent penetration of ink; and/or
wherein the color pattern (33) is configured to include a plurality of color components that exhibit different changes for one degradation factor.

11. The pre-cured tread (170) according to any one of claims 8 to 10, wherein the degradation factor includes at least one of heat, oxygen, water, ultraviolet light, and ozone; and/or
wherein the at least one color pattern (33) comprises a plurality of color patterns (33) printed on the medium layer (37).

## Patentansprüche

1. Reparaturflicken (70) zur Anbringung an einem Reifen (30), wobei der Reparaturflicken (70) Folgendes umfasst:
einen Verstärkungsabschnitt (71), der mit dem Reifen (30) verklebt werden soll, um den Reifen (30) zu verstärken,
**dadurch gekennzeichnet, dass**
der Reparaturflicken ferner Folgendes umfasst:
einen Indikatorabschnitt, der Farbkomponenten aufweist, die von einer Verschleißzustandsbestimmungseinrichtung extrahiert werden, die ein Verschleißzustandsmodell und eine Farbänderungsdatenbank zum Bestimmen eines Verschleißzustands des Reifens beinhaltet, wobei
der Indikatorabschnitt (31a) eine Mittelschicht (37), die eine Kennung (32) und mindestens ein Farbmuster (33), das eine Vielzahl von Farben an verschiedenen Positionen aufgebracht aufweist, darauf aufgedruckt aufweist, wobei die Vielzahl von Farben unterschiedliche Empfindlichkeiten gegenüber einem Verschleißfaktor aufweisen.

2. Reparaturflicken (70) nach Anspruch 1, wobei der Indikatorabschnitt (31a) ferner eine Schutzschicht (38) umfasst, die auf einer vom Reifen (30) abgewandten Seite der Mittelschicht (37) bereitgestellt ist und dazu gestaltet ist, die Durchlässigkeit von mindestens ultraviolettem Licht zu hemmen.

3. Reparaturflicken (70) nach Anspruch 1 oder 2, wobei der Verstärkungsabschnitt (71) eine Haftschicht (72) umfasst, die auf einer dem Reifen (30) näheren Seite der Mittelschicht (37) bereitgestellt ist und mit einem Klebstoff am Reifen (30) angeklebt werden soll.

4. Reparaturflicken (70) nach einem der Ansprüche 1 bis 3, wobei der Indikatorabschnitt (31a) ferner eine Penetrationsschutzschicht (36) umfasst, die zwischen der Mittelschicht (37) und dem Verstärkungsabschnitt (71) bereitgestellt und dazu gestaltet ist, ein Eindringen von Tinte zu verhindern.

5. Reparaturflicken (70) nach einem der Ansprüche 1 bis 4, wobei das Farbmuster (33) dazu gestaltet ist, eine Vielzahl von Farbkomponenten zu beinhalten, die für einen Verschleißfaktor unterschiedliche Veränderungen aufweisen.

6. Reparaturflicken (70) nach einem der Ansprüche 1 bis 5, wobei der Verschleißfaktor mindestens eines von Wärme, Sauerstoff, Wasser, ultraviolettem Licht und Ozon beinhaltet.

7. Reparaturflicken (70) nach einem der Ansprüche 1 bis 6, wobei das mindestens eine Farbmuster (33) eine Vielzahl von auf die Mittelschicht (37) gedruckten Farbmustern umfasst.

8. Vorgehärtete Lauffläche (170), die Folgendes umfasst:
einen Indikatorabschnitt (31a),
**dadurch gekennzeichnet, dass**
die vorgehärtete Lauffläche (170) ferner Folgendes umfasst:
einen Hauptkörper (171), der aus vulkanisiertem Kopfgummi gestaltet ist, wobei der Indikatorabschnitt (31a) Farbkomponenten aufweist, die extrahiert werden sollen durch
eine Verschleißzustandsbestimmungseinrichtung (10), die ein Verschleißzustandsmodell und eine Farbänderungsdatenbank (121) zum Bestimmen eines Verschleißzustands eines Reifens (30) beinhaltet; und
wobei
der Indikatorabschnitt (31a) eine Mittelschicht (37), die eine Kennung (32) und mindestens ein Farbmuster (33), das eine Vielzahl von Farben an verschiedenen Positionen aufgebracht aufweist, darauf aufgedruckt aufweist, wobei die Vielzahl von Farben unterschiedliche Empfindlichkeiten gegenüber einem Verschleißfaktor aufweisen.

9. Vorgehärtete Lauffläche (170) nach Anspruch 8, wobei der Indikatorabschnitt (31a) ferner eine Schutzschicht (38) umfasst, die auf einer vom Reifen (30) abgewandten Seite der Mittelschicht (37) bereitgestellt ist und dazu gestaltet ist, die Durchlässigkeit von mindestens ultraviolettem Licht zu hemmen.

10. Vorgehärtete Lauffläche (170) nach Anspruch 8 oder 9, wobei der Indikatorabschnitt (31a) ferner eine Penetrationsschutzschicht (36) umfasst, die zwischen der Mittelschicht (37) und dem Hauptkörper (171) bereitgestellt und dazu gestaltet ist, ein Eindringen von Tinte zu verhindern; und/oder
wobei das Farbmuster (33) dazu gestaltet ist, eine Vielzahl von Farbkomponenten zu beinhalten, die für einen Verschleißfaktor unterschiedliche Veränderungen aufweisen.

11. Vorgehärtete Lauffläche (170) nach einem der Ansprüche 8 bis 10, wobei der Verschleißfaktor mindestens eines von Wärme, Sauerstoff, Wasser, ultraviolettem Licht und Ozon beinhaltet; und/oder
wobei das mindestens eine Farbmuster (33) eine Vielzahl von auf die Mittelschicht (37) gedruckten Farbmustern (33) umfasst.

## Revendications

1. Patch de réparation (70) destiné à être attaché à un pneu (30), le patch de réparation (70) comprenant :
une portion de renfort (71) à coller au pneu (30) pour renforcer le pneu (30),
**caractérisé en ce que**
le patch de réparation comprend en outre :
une portion d'indicateur présentant des composants de couleur à extraire par un appareil de détermination d'état de dégradation qui inclut un modèle d'état de dégradation et une base de données de changement de couleur pour déterminer un état de dégradation du pneu, dans lequel
la portion d'indicateur (31a) comprend une couche intermédiaire (37) sur laquelle est imprimé un identifiant (32) et au moins un motif de couleur (33) présentant une pluralité de couleurs appliquées à différentes positions, la pluralité de couleurs présentant des sensibilités différentes à un facteur de dégradation.

2. Patch de réparation (70) selon la revendication 1, dans lequel la portion d'indicateur (31a) comprend en outre une couche protectrice (38) fournie sur un côté de la couche intermédiaire (37) à l'écart du pneu (30) et configurée pour empêcher la transmission d'au moins de la lumière ultraviolette.

3. Patch de réparation (70) selon la revendication 1 ou 2, dans lequel la portion de renforcement (71) comprend une couche d'adhérence (72) qui est fournie sur un côté de la couche intermédiaire (37) plus proche du pneu (30) et doit être collée au pneu (30) par un adhésif.

4. Patch de réparation (70) selon l'une quelconque des revendications 1 à 3, dans lequel la portion d'indicateur (31a) comprend en outre une couche de prévention de pénétration (36) fournie entre la couche intermédiaire (37) et la portion de renforcement (71) et configurée pour empêcher la pénétration d'encre.

5. Patch de réparation (70) selon l'une quelconque des revendications 1 à 4, dans lequel le motif de couleur (33) est configuré pour inclure une pluralité de composants de couleur qui exposent différents changements pour un facteur de dégradation.

6. Patch de réparation (70) selon l'une quelconque des revendications 1 à 5, dans lequel le facteur de dégradation inclut au moins un parmi de la chaleur, de l'oxygène, de l'eau, de la lumière ultraviolette et de l'ozone.

7. Patch de réparation (70) selon l'une quelconque des revendications 1 à 6, dans lequel l'au moins un motif de couleur (33) comprend une pluralité de motifs de couleur imprimés sur la couche intermédiaire (37).

8. Bande de roulement pré-durcie (170) comprenant :
une portion d'indicateur (31a),
**caractérisée en ce que**
la bande de roulement pré-durcie (170) comprend en outre :
un corps principal (171) configuré par une tête en caoutchouc vulcanisé, dans laquelle la portion d'indicateur (31a) présente des composants de couleur à extraire par
un appareil de détermination d'état de dégradation (10) qui inclut un modèle d'état de dégradation et une base de données de changement de couleur (121) pour déterminer un état de dégradation d'un pneu (30) ; et
dans laquelle
la portion d'indicateur (31a) comprend une couche intermédiaire (37) sur laquelle est imprimé un identifiant (32) et au moins un motif de couleur (33) présentant une pluralité de couleurs appliquées à différentes positions, la pluralité de couleurs présentant des sensibilités différentes à un facteur de dégradation.

9. Bande de roulement pré-durcie (170) selon la revendication 8, dans lequel la portion d'indicateur (31a) comprend en outre une couche protectrice (38) fournie sur un côté de la couche intermédiaire (37) à l'écart du pneu (30) et configurée pour empêcher la transmission d'au moins de la lumière ultraviolette.

10. Bande de roulement pré-durcie (170) selon la revendication 8 ou 9, dans lequel la portion d'indicateur (31a) comprend en outre une couche de prévention de pénétration (36) fournie entre la couche intermédiaire (37) et le corps de renforcement (171) et configurée pour empêcher la pénétration d'encre ; et/ou
dans laquelle le motif de couleur (33) est configuré pour inclure une pluralité de composants de couleur qui exposent différents changements pour un facteur de dégradation.

11. Bande de roulement pré-durcie (170) selon l'une quelconque des revendications 8 à 10, dans laquelle le facteur de dégradation inclut au moins un parmi de la chaleur, de l'oxygène, de l'eau, de la lumière ultraviolette et de l'ozone ; et/ou
dans laquelle l'au moins un motif de couleur (33) comprend une pluralité de motifs de couleur (33) imprimés sur la couche intermédiaire (37).
